(19) Europäisches Patentamt
European Patent Office
Office européen des brevets

(11) **EP 4 098 183 A1**

(12) **EUROPEAN PATENT APPLICATION**

(43) Date of publication:
**07.12.2022 Bulletin 2022/49**

(21) Application number: **21177428.6**

(22) Date of filing: **02.06.2021**

(51) International Patent Classification (IPC):
**A61B 5/0225** (2006.01)    **A61B 5/00** (2006.01)

(52) Cooperative Patent Classification (CPC):
**A61B 5/02255; A61B 5/6803;** A61B 2562/043;
A61B 2562/063

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **MUEHLSTEFF, Jens**
**5656 AE Eindhoven (NL)**
• **WIJSHOFF, Ralph Wilhelm Christianus Gemma Rosa**
**5656 AE Eindhoven (NL)**
• **WANG, Wenjin**
**5656 AE Eindhoven (NL)**

(74) Representative: **Philips Intellectual Property & Standards**
**High Tech Campus 52**
**5656 AG Eindhoven (NL)**

(54) **BLOOD PRESSURE DETECTION**

(57)    A system and method for non-contact or remote monitoring of blood pressure changes of a subject. Embodiments are based on using a headgear apparatus with a skin interface module, arranged when worn to apply a pre-defined non-uniform pressure profile to a region of skin. The system includes optical or electromagnetic sensing equipment, e.g. a camera, arranged with a field of view which covers the skin interface module and arranged to obtain optical sensing data for the skin region being applied with the pressure profile. From the optical sensing data, remote PPG sensor readings can be obtained for a plurality of skin contact locations being applied with different respective pressures. Blood pressure changes can be detected based on changes in the distribution of PPG signal (or derived pulse amplitude signal) readings across the plurality of skin contact locations.

FIG. 1

EP 4 098 183 A1

**Description**

FIELD OF THE INVENTION

This invention relates to a system for detection and monitoring of blood pressure.

BACKGROUND OF THE INVENTION

Arterial blood pressure (ABP) is the pressure exerted by blood on the wall of an arterial blood vessel. Due to the cyclical pumping of the heart, blood flow is pulsatile in the arteries. Blood pressure values are typically measured as a set which comprises the systolic peak value, mean arterial pressure and the diastolic minimum value.

In high acuity healthcare settings, invasive ABP (iABP) measurements using catheters are common. This approach permits tracking of the complete ABP waveform with a very high temporal and pressure resolution. This is often required during surgery and in intensive care units (ICU). Invasive ABP carries a risk of infection and complications such as bleeding and perfusion deficit. It also requires trained personnel for use. It is therefore only suitable for high acuity settings. However, iABP is the "gold-standard" for blood pressure monitoring.

A noninvasive alternative is cuff-based ABP measurement using the oscillometric or auscultation method. The oscillometric method is based on controlled variation of an external cuff pressure on an artery (typically the brachial or radial artery) over time. The changing external pressure results in a change of the transmural pressure (defined as the difference between the internal pressure in the artery and the applied cuff pressure). The artery responds with artery diameter changes dependent on the applied pressure. These diameter changes are detected as cuff pressure oscillations which follow a characteristic pattern used to infer blood pressure.

Cuff-based measurement provides only intermittent measurements of systolic blood pressure (SBP), diastolic blood pressure (DBP) and mean arterial pressure (MAP). Measurement intervals range from 2-3 minutes in an operating room setting, typically 15 minutes in intensive care setting, and up to hours in a general ward. Therefore, cuff-based measurements may miss sudden, critical blood pressure changes. Furthermore, the low temporal resolution of measurements also makes cuff-based measurement a problematic choice for use in physiological monitoring, or as an input to improve gating, during MRI or CT scanning. Furthermore, cuff-based measurement is uncomfortable and disturbs sleep.

There is a need for a system and method which permits remote and continuous monitoring of blood pressure, and detection of changes in blood pressure, and which is suitable for use in both high and low acuity settings such as the emergency department (ED), ICU and general ward, and during medical procedures (CT, MRI), and at home.

SUMMARY OF THE INVENTION

The invention is defined by the claims.

In a different technical field, there is known technology for performing remote optical, e.g. camera-based, vital sign detection. It has been shown that pulse rate, pulse amplitudes and SpO2 can be remotely detected. Embodiments of the present invention are based on adaptation of these technologies for use in monitoring blood pressure.

According to examples in accordance with an aspect of the invention, there is provided a system comprising: a head-mountable apparatus arranged, when mounted to a user's head, to engage with a plurality of tissue contact locations across a tissue area of the user, and to apply a different respective contact pressure to each of the plurality of tissue-contact locations; an optical sensing device adapted to detect optical emissions from the tissue area of the user and output optical sensing data based thereon; and a processor, adapted to: receive the optical sensing data; derive a photoplethysmography (PPG) signal for each of the plurality of tissue-contact locations based on the optical sensing data; detect a blood pressure of the user, or a change in blood pressure of the user, based on the PPG signals for the plurality of tissue contact locations and based on the respective contact pressures at each of the tissue contact locations.

Embodiments of the invention are based on use of a dedicated pressure-application structure to apply a non-uniform pressure profile across a tissue area, and use of remote PPG sensing to detect blood volume oscillations across different locations within the area. The non-uniform pressure results in a non-uniform transmural pressure within the blood vessels of the tissue area. In effect, the invention replaces the time-varying pressure profile applied by a blood pressure cuff with a spatially varying pressure profile across an area of tissue of the user. Thus multiple pressures are applied simultaneously. Remote PPG allows for blood volume oscillations to be remotely measured, from which pulse amplitude can be detected. By measuring how pulse amplitude varies with applied pressure, blood pressure information can be derived.

The defined system allows for instantaneous (beat-to-beat) detection of blood pressure changes, including the ability to instantaneously discriminate an increase or a decrease in blood pressure (as will be explained further below). If a calibration is performed, a quantified blood pressure measurement can be achieved.

Preferably, the processor monitors the PPG signals over time, so that changes in the signals can be detected.

The processor may be further adapted to generate a data output indicative of the detection. The processor may generate an output for communication to a user interface for causing the user interface to display an indication of the detection.

The processor may determine a measure or metric directly or indirectly indicative of the blood pressure, or indicative of a change in blood pressure. In some embodiments, the processor detects occurrence of a change in blood pressure without determining a quantitative measure of blood pressure.

The area of the user's tissue used for the detection is preferably on the forehead, where the tissue surface is relatively homogeneous.

In some embodiments, the processor may be adapted to determine a pulse amplitude signal for each of the different tissue contact locations, and wherein detecting the blood pressure or change in blood pressure is based on the pulse amplitude signal for each of the tissue-contact locations.

The pulse amplitude may be simply the amplitude of the PPG signal, since the PPG signal is indicative of blood volume oscillations. Alternatively, the pulse amplitude signal may be the amplitude of the PPG signal normalized (divided) by the baseline of the PPG signal, where the baseline of the PPG signal can be obtained by applying a low-pass filter with a cut-off at e.g. 0.5 Hz or less.

In some embodiments, the processor may be adapted to: identify which of the plurality of tissue contact locations has a highest detected pulse amplitude; detect a change in which of the tissue contact locations has a highest detected pulse amplitude; and detect occurrence of a change in blood pressure based on said detected change in the tissue contact location for which the pulse amplitude is highest.

As noted above, the different tissue contact pressures result in differing transmural pressures in the underlying blood vessels. Thus, depending upon the arterial blood pressure, the maximum detectable pulse amplitude at a given moment in time will occur in a blood vessel location with a different transmural pressure. By detecting a change in the location at which the maximum pulse amplitude is detected, a change in arterial blood pressure is detectable.

In some embodiments, the processor may be adapted to: detect a change in tissue contact location for which the pulse amplitude is highest from a first location to a second location, and if the contact pressure of the second location is higher than the first location, detect occurrence of an increase in blood pressure, and if the contact pressure of the second location is lower than the first location, detect occurrence of a decrease in blood pressure.

An increasing blood pressure will mean that the maximum detected pulse amplitude will change to location with a higher externally applied pressure, and vice versa.

In some embodiments, a quantitative measure of blood pressure may be determined. This may be based on a quantitative measure of the contact pressure between the tissue and the apparatus for each of the tissue contact locations. Additionally or alternatively, it may be based on a reference blood pressure measurement obtained at a start of a monitoring period.

Likewise, a quantitative measure of a change in the blood pressure can also be determined provided the contact pressures at the plurality of locations are known.

In some embodiments, the system may further comprise a light source adapted to direct a light output toward said tissue area of the user.

This allows for more accurate or reliable PPG measurements, since the spectral properties and intensity of the light can be controlled. However, a natural light source can instead be used, meaning that provision of an artificial illumination source is not essential.

In preferred embodiments, the optical sensing device may be physically separate from the head-mountable apparatus. This permits remote monitoring of the blood pressure.

The optical sensing device may be a camera in some embodiments.

In some embodiments, the head-mountable apparatus may comprise a tissue contact section arranged to engage with said tissue area of the user to apply the different contact pressures to each of the plurality of tissue contact locations.

The tissue contact section may incorporate an optical window permitting optical communication with the plurality of tissue contact locations by the optical sensing device.

The optical window is an optically transmissive element. It may for example be a sheet or laminar element. It may be optically transparent.

In some embodiments, the optical window may be arranged to engage with said tissue area of the user to apply the different contact pressures to each of the plurality of tissue contact locations.

In some embodiments, the optical window may have a contact surface adapted to engage with said tissue area of the user, and wherein the optical window has a non-uniform thickness (t) in a dimension out-of-plane of said contact surface.

The head-mounted apparatus may be adapted to urge the optical window against the area of the user's tissue with a force which is symmetrically or homogeneously distributed across the contact surface area. As such, the different thicknesses result in non-uniform pressure being exerted across the tissue contact area. This provides the different contact pressures at the plurality of tissue contact locations.

The thickness of the window may vary along a linear direction or dimension of the optical window.

In some embodiments, the optical window may have a continuously varying thickness (t) across at least a portion of the window area. This provide a continuous pressure gradient. The plurality of locations may be a set of locations along this pressure gradient, for instance at regular spatial intervals.

In some embodiments, the optical window may comprise a plurality of window area sections, each window area section having a different respective thickness. Each discrete window section has a uniform thickness across its respective area, so that the thickness varies discontinuously across the window.

In some embodiments, the optical window may feature a plurality of optically visible markings disposed on a surface facing away from the user when the apparatus is mounted to the user's head, and each optically visible marking at a location corresponding to a different window thickness (i.e. a location corresponding to a different contact pressure).

The marking may be readable by the optical sensing apparatus, e.g. a readable code, to permit the optical sensing apparatus to (spatially) identify the different tissue contact locations. The markings may provide an indication of the relative pressure applied at the corresponding location.

For example, where the optical window comprises a plurality of window sections of different thicknesses, each window section may feature one of the respective optically visible markings.

In a preferred set of embodiments, the tissue-contact section may consist of the optical window alone.

In some embodiments, the optical window may include a pressure-responsive material having an optical characteristic which is adapted to vary as a function of pressure. The optical window may include the pressure responsive material at least at each of the different skin contact locations. For example, there may be an area of the pressure responsive material overlying at least a region of the skin contact location. This provides a means for optically identifying the differing pressures at the different skin contact locations.

For example, each optical window section may include a sub-region which comprises the pressure-responsive material, and a sub-region which is optically transparent (for obtaining the optical sensing data).

In some embodiments, the head-mountable apparatus, when mounted to the head of the user, may be arranged to hold the tissue-contact section against the tissue of the user with an application force, and wherein the apparatus includes actuation means for varying said application force.

In some embodiments, the apparatus may comprise a headband for holding the tissue contact section against a forehead of the user.

In some embodiments, the apparatus may include one or more actuator elements arranged to be disposed between the headband and the user's head when the apparatus is mounted to the user. The actuation of the actuator elements may be adapted to vary a separation distance of the headband from the head.

The one or more actuator elements may be balloons with a controllable inflation level.

In some embodiments, the actuation means may comprise one or more electroactive polymer (EAP) actuator elements disposed at each of a first and second side of the skin contact section.

An EAP actuator element comprises an EAP material which is adapted to exhibit a deformation in response to stimulation by an electrical current or voltage. The extent of deformation is dependent on the magnitude of the voltage or the amplitude of the current. These may be disposed for example between a headband of the apparatus and the skin directly adjacent each of the first and second end of the skin contact section. This permits varying the baseline application force of the tissue contact section on the user's tissue. The actuation level of two actuator elements can be varied symmetrically or asymmetrically. If varied asymmetrically, this permits changing of (a steepness of) a pressure gradient across the plurality of tissue contact locations.

Examples in accordance with a further aspect of the invention provide a method, comprising: simultaneously applying a different respective contact pressure to each of a plurality of tissue-contact locations across a tissue area of a user; detecting optical emissions from said tissue area of the user and generating optical sensing data based thereon; deriving a photoplethysmography (PPG) signal for each of the plurality of tissue-contact locations based on the optical sensing data; and detecting a blood pressure of the user, or a change in blood pressure of the user, based on the PPG signals for the plurality of tissue contact locations and based on the respective contact pressures at each of the tissue contact locations. The second, third and fourth steps of the above method may be implemented by a computer or a processor in some examples.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows an example system according to one or more embodiments;

Fig. 2 shows an example head-mountable apparatus according to one or more embodiments;

Fig. 3 illustrates an example optical window for applying a non-uniform pressure profile according to one or more embodiments,

Fig. 4 illustrates an example optical window having a stepped thickness for applying the non-uniform pressure profile;

Fig. 5 shows an example optical window having a smoothly inclined thickness for applying the non-uniform pressure profile;

Fig. 6 shows an example optical window having a plurality of marked window sections;

Fig. 7 shows an example head-mountable apparatus with actuator means for adjusting an application pressure;

Fig. 8 shows a further example head-mountable apparatus with actuator means for adjusting application pressure; and

Fig. 9 shows an example method in accordance with one or more embodiments.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for non-contact or remote monitoring of blood pressure changes of a subject. This is based on using a headgear apparatus with a skin interface module arranged, when worn, to apply a pre-defined non-uniform pressure profile to a region of skin, preferably on the forehead. The system includes optical or electromagnetic sensing equipment, e.g. a camera, arranged with a field of view which covers the skin interface module and arranged to obtain optical sensing data for the skin region being applied with the pressure profile. From the optical sensing data, remote PPG sensor readings can be obtained for a plurality of skin contact locations being applied with different respective pressures. Blood pressure changes can be detected based on changes in the distribution of PPG signal (or derived pulse amplitude signal) readings across the plurality of skin contact locations.

The method effectively replaces the time-varying pressure profile applied to an arm during an oscillometric blood pressure measurement with instead a spatially varying pressure profile applied to a region of skin. It further replaces contact-based volume oscillation measurement used in the oscillometric technique with instead remote-PPG based volume oscillation measurement.

It is noted that although embodiments of the invention are described using a head-mountable apparatus for applying a pressure profile to a skin region of the forehead, the invention is also applicable to a pressure profile being applied to other regions of the body. Thus, in all embodiments described herein, the head-mountable apparatus could be replaced with a body-mountable apparatus, for mounting to any suitable region of the body, and arranged, when mounted to the body, to engage with a plurality of tissue contact locations across a tissue area of the user, and to apply a different respective contact pressure to each of the plurality of tissue-contact locations.

As noted above, the gold standard for blood pressure measurement is invasive blood pressure measurement. However, invasive blood pressure measurement techniques have a number of disadvantages: the hardware is difficult to apply, meaning the technique is limited to high acuity settings; qualified personnel are required; the hardware takes a significant length of time to apply; the setup is complex (transducer, infusion solution); there is risk of infection and contamination due to the invasive nature of the technique; and there is a risk of bleeding and clotting.

As noted above, the oscillometric blood pressure measurement technique is based on applying an external cuff pressure on an artery (typically the brachial or radial artery) which varies in a characteristic pattern over time. The oscillometric technique has a number of disadvantages: attaching a blood pressure cuff is cumbersome; the blood pressure measurement has a fairly long duration (20s - 60s); it requires use of a complex pneumatic and pressure transducer; it does not permit instant and continuous blood pressure monitoring (due to the need to apply a pressure sweep to obtain a single pressure measurement); temporal resolution is poor due to the long time duration for pressure measurements, meaning critical blood pressure changes can be missed.

Separately to the field of blood pressure monitoring, there is known technology for remote (optical based) vital sign detection. For example, this can be done with a camera and suitable analysis algorithms. It has been shown that pulse rate, pulse amplitudes and $SpO_2$ can be remotely detected. In this regard, reference is made for example to the following patent documents which detail example remote PPG sensing apparatuses suitable for use in embodiments of the present invention: US Patent No. 10290104 B2, US Patent No. 9980658 B2, US Patent No. 10242441 B2, and US Patent No. 10818010 B2. Reference is also made to the following papers which also provide details of example PPG sensing systems suitable for use in embodiments of the present invention:

Moço A, Verkruysse W., Pulse oximetry based on photoplethysmography imaging with red and green light: Calibratability and challenges. J Clin Monit Comput. 2021 Feb;35(1):123-133.

Van Gastel, M. et al, Data-Driven Calibration Estimation for Robust Remote Pulse-Oximetry. Appl. Sci. 2019, 9, 3857.

Mark van Gastel et al, "Near-continuous non-contact cardiac pulse monitoring in a neonatal intensive care unit in near darkness," Proc. SPIE 10501, Optical Diagnostics and Sensing XVIII: Toward Point-of-Care Diagnostics, 1050114 (20 February 2018).

Verkruysse, Wim PhD et al, Calibration of Contactless Pulse Oximetry, Anesthesia & Analgesia: January 2017 - Volume 124 - Issue 1 - p 136-145.

Aarts LA, et al. Non-contact heart rate monitoring utilizing camera photoplethysmography in the neonatal intensive care unit - a pilot study. Early Hum Dev. 2013 Dec;89(12):943-8.

However, there are currently no known techniques for remote blood pressure monitoring. Embodiments of the present invention aim to address this problem by providing a remote blood pressure monitoring solution which is based on optical sensing, enabling remote detection and quantification of pulse amplitudes for a plurality of skin areas simultaneously.

Embodiments of the present invention allow for remote blood pressure monitoring, which can be used for example for assessment of hemodynamic states, and changes in patient status both in clinical settings (including non-hospital settings), and optionally in the home environment.

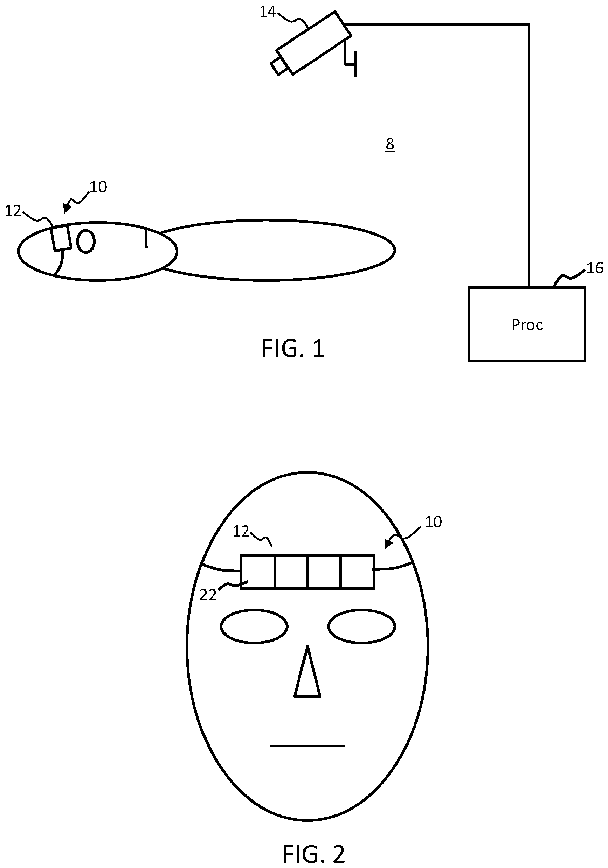

Fig. 1 shows an example system 8 in accordance with one or more embodiments.

The system comprises a body-mountable apparatus 10 which is arranged, when mounted to a pre-defined location of a user's body, to engage with a plurality of tissue contact locations across a tissue area of the user, and to apply a different respective contact pressure to each of the plurality of tissue-contact locations. In the example illustrated in Fig. 1, the body-mountable apparatus is a head-mountable apparatus arranged, when mounted to the user's head, to engage with a plurality of tissue contact locations across a tissue area of the user's head. In the illustrated example, the apparatus 10 is arranged to engage with a tissue area on the forehead of the user.

The system further comprises an optical sensing apparatus or 14 adapted to detect optical emissions from the tissue area of the user and output optical sensing data based thereon. When the apparatus is worn, the plurality of tissue contact locations are optically accessible to the optical sensing device. The optical sensing device may be a camera in some examples. Alternatively, it may be a different type of light-sensitive apparatus, e.g. non-image based optical sensing. Optical includes visible light, but may also include non-visible light spectra such as infrared or ultraviolet. It may in further examples be sensitive to other regions of the electromagnetic spectrum.

The system 8 further comprises a processor 16, adapted to: receive the optical sensing data from the optical sensing device; derive a photoplethysmography (PPG) signal for each of the plurality of tissue-contact locations based on the optical sensing data; and detect a blood pressure of the user, or a change in blood pressure of the user, based on the PPG signals for the plurality of tissue contact locations and based on the respective contact pressures at each of the tissue contact locations. The PPG signal allows for pulsatility at each tissue contact location to be derived, as will be explained below.

The processor 16 may be further adapted to generate a data output indicative of the detection. The processor may generate an output for communication to a user interface for causing the user interface to display an indication of the detection.

In the example illustrated in Fig. 1, the subject is located in a position within the field of view of the optical sensing device 14, but with the optical sensing device spaced from the subject. This allows for remote blood pressure monitoring. Although Fig. 1 shows the processor 16 positioned close to the optical sensing device, this is for schematic illustration only. The processor may be provided remote from the optical sensing apparatus, and in communication with the optical sensing apparatus via a wireless communication link, or an Internet link. Alternatively, the processor may be provided with the optical sensing apparatus, and in communication with the optical sensing apparatus via a wired communication link.

In one aspect of the invention, the whole system 8 may be provided. However, in a further aspect, just the body-mountable apparatus 10 and the optical sensing device 14 might be provided as a sub-system. This provides the necessary hardware for acquiring the measurement data. This might then be coupled with an existing processor 16 to derive the blood pressure measurement information. In another aspect, just the processor might be provided alone, arranged to receive the optical sensing data acquired by the optical sensing device, either in real time with the data acquisition, or from a datastore where the data has been cached.

The defined system allows for instantaneous (beat-to-beat) detection of blood pressure changes, including the ability to instantaneously discriminate an increase or a decrease in blood pressure (as will be explained below). If a calibration is performed, a quantified blood pressure measurement can be achieved.

Preferably, the processor monitors the PPG signals over time, so that changes in the signals can be detected.

The processor may determine a measure or metric directly or indirectly indicative of the blood pressure, or indicative of a change in blood pressure. In some embodiments, the processor 16 detects occurrence of a change in blood pressure without determining a quantitative measure of blood pressure.

The provided system allows for remote PPG signal pulse amplitudes to be detected at different transmural pressures simultaneously across a tissue region. A benefit of using the forehead is the relative homogeneity of tissue in the region. The optical sensing device is operable to detect the blood pulsatile components across the different contact locations at any moment in time. The relative amplitudes between the different locations (with different externally applied pressures) provides information on the change of blood pressure.

In some examples, a reference blood pressure measurement may be obtained at a start of a monitoring period, for example using an oscillometric technique or another technique. This provides a baseline or reference level. The processor may be operable to derive an indication of a change in blood pressure (as will be described below). Based on this detected measure of blood pressure change since a start of monitoring period, and on the reference blood pressure measurement acquired at the start of the monitoring period, a quantitative measure of blood pressure at any given time during the monitoring period can be determined. Thus the system is operable to determine and output a real-time quantitative measure of blood pressure at any given time.

A calibration process may be performed at a start of a monitoring period comprising the processor 16 receiving an indication of a reference blood pressure measurement. This may be received via a user interface device for example. Quantitative blood pressure measurements may be derived based on this calibration. The calibration process may further comprise receiving or deriving an indication of the pressure being applied at each tissue contact location. This may be based on sensing optical characteristics of a pressure-responsive material comprised by a tissue-contact section of the head-mountable apparatus (as will be explained further below).

The processor 16 may be comprised by a standalone processing unit, or may for example be a processor of a computing unit such as a mobile computing device, e.g. a smartphone or tablet computer.

The head-mountable apparatus 10 may comprise a tissue contact section or module 12 arranged to engage with said tissue area of the user to apply the different contact pressures to each of the plurality of tissue contact locations. Fig. 2 shows an example.

The tissue contact section 12 preferably incorporates an optical window 22 permitting optical communication with the plurality of tissue contact locations by the optical sensing device 14. The optical window is an optically transmissive element. It may for example be a sheet or laminar element. It may be optically transparent.

The body-mountable apparatus 10 in the illustrated example further comprises a band or strap for holding the tissue contact section 12 against the skin. It may however comprise any other type of retaining means for holding the tissue contact section against the skin.

In some embodiments, the optical window 22 is arranged to engage with said tissue area of the user to apply the different contact pressures to each of the plurality of tissue contact locations.

Embodiments employ use of so-called remote PPG sensing, by which PPG measurements for a tissue area can be obtained in a non-contact manner using optical sensing data from, for example, a camera. Methods for implementing remote PPG sensing, suitable for use in embodiments of the present invention, are described for example in the following documents: US Patent No. 10290104 B2, US Patent No. 9980658 B2, US Patent No. 10242441 B2, and US Patent No. 10818010 B2.

Preferably the processor 16 is adapted to determine a pulse amplitude signal for each of the different tissue contact locations, and wherein detecting the blood pressure or change in blood pressure is based on the pulse amplitude signal for each of the tissue-contact locations.

The pulse amplitude may be simply the amplitude of the PPG signal, since the PPG signal is indicative of blood volume oscillations. Alternatively, the pulse amplitude signal may be the amplitude of the PPG signal normalized by (divided by) the baseline of the PPG signal, where the baseline of the PPG signal can be obtained by applying a low-pass filter with a cut-off at e.g. 0.5 Hz or less.

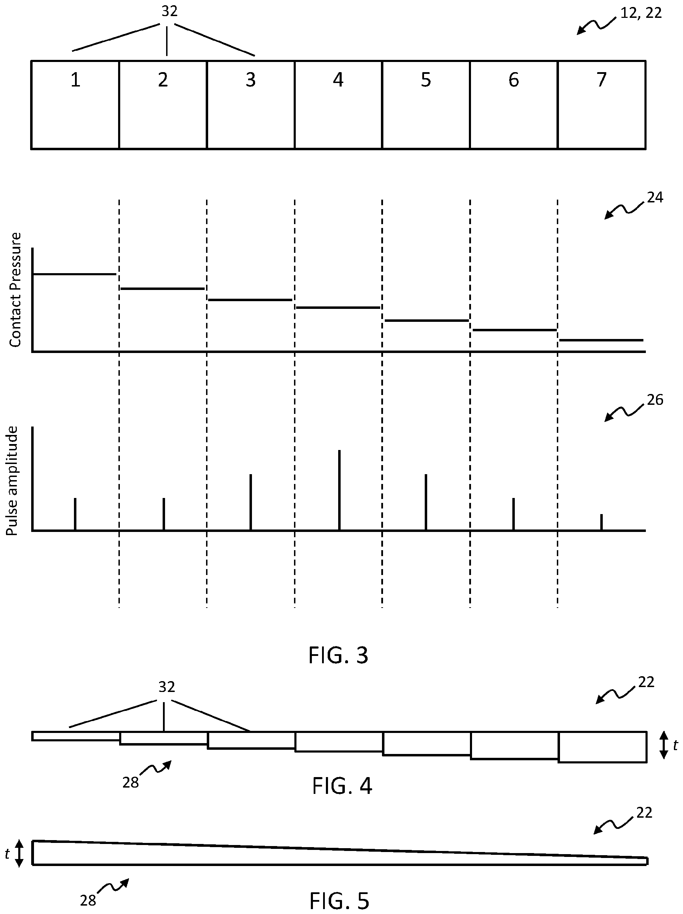

The basic principle for measuring blood pressure and variations of blood pressure is illustrated schematically in Fig. 3. At the top of Fig. 3 is shown a schematic illustration of an example tissue contact section 12 comprising an optical window 22. In this example, the optical window is designed to apply the non-uniform pressure profile to the tissue region. In this example, the optical window is divided into a plurality of different window sections 32, wherein, when the apparatus is worn, each different section is arranged to apply a different respective pressure to the area of tissue located beneath it. The area of skin underneath each window section 32 corresponds to one of the tissue contact locations referred to previously. In the illustrated example, there are seven window sections, but this is illustrative only and any number of window sections and/or tissue contact locations can be provided.

The graph 24 in the middle of Fig. 3 schematically illustrates the different contact pressures applied to tissue contact regions beneath each respective window section 32. Due to the different externally applied pressures, different transmural pressures are realized at each location.

The graph 26 at the bottom of Fig. 3 shows remotely detected (by an optical sensing apparatus such as a camera system) PPG signal amplitudes (pulse amplitudes). In the illustrated example, the maximum pulse amplitude is detected at window section 4.

According to embodiments, detection of a change in blood pressure of a subject may comprise detecting a change in the distribution 26 of pulse amplitude measurements between the different tissue contact locations having different externally applied pressures.

In particular, in accordance with one or more embodiments, the processor 16 may be adapted to: identify which of the plurality of tissue contact locations has a highest detected pulse amplitude; detect a change in which of the tissue contact locations has a highest detected pulse amplitude; and detect occurrence of a change in blood pressure based on said detected change in the tissue contact location for which the pulse amplitude is highest.

For example, the pulse amplitude distribution or profile shown in Fig. 3 may correspond to a starting state for the subject. At a later time, the pulse amplitude profile may change so that the maximum pulse amplitude is detected at window 6. In this case, the maximum pulse amplitude has moved to a tissue contact location with a lower externally applied pressure. This indicates a blood pressure decrease. By contrast, at a later time, the maximum pulse amplitude might move to the tissue contact location corresponding to window 2. In this case, the maximum pulse amplitude has moved to a tissue contact location with a higher externally applied pressure, indicating a blood pressure increase.

Thus, in accordance with one or more embodiments, the processor may be adapted, in at least one control mode, to: detect a change in tissue contact location for which the pulse amplitude is highest from a first location to a second location, and if the contact pressure of the second location is higher than the first location, detect occurrence of an increase in blood pressure, and if the contact pressure of the second location is lower than the first location, detect occurrence of a decrease in blood pressure.

This method enables detection of relative changes in blood pressure: in particular it can be detected when blood pressure has increased, and when blood pressure has decreased.

If a quantitative applied pressure difference between the different tissue contact locations is known, then a quantitative measure of blood pressure change can be derived. For example, it may be known that consecutive window sections 32 differ in the respective pressure that they apply to tissue underlying them by amount $\Delta p$. Then, if the maximum pulse amplitude moves from window section $a$ to window section $b$, the pressure difference between window $a$ and window $b$ can be determined, and a quantitative measure of the blood pressure change determined.

In particular, the blood pressure change, $\Delta$BP, may be estimated as:

$$\Delta\text{BP} = (\text{SectionNumberNewMax} - \text{SectionNumberOldMax}) * \Delta p$$

where SectionNumberNewMax is the number of the window section 32 with the new maximum amplitude, SectionNumberOldMax is the number of the window section of the previous maximum amplitude, and $\Delta p$ is the change in the applied pressure from one window section to a neighboring window section. In the above equation, $\Delta p$ is assumed to be negative between a first section of lower section number and a neighboring second section of higher section number. In other words, if for example the maximum pulse amplitude location moves from window section 4 to window section 6, $\Delta$BP = (6 - 4) * $\Delta p$ = 2 * $\Delta p$, indicating a net negative blood pressure change (decrease in pressure). If the maximum pulse amplitude moves from section 4 to section 2, $\Delta$BP = (2 - 4) * $\Delta p$ = -2 * $\Delta p$, indicating a net positive blood pressure a change (a pressure rise).

Furthermore, as mentioned above, if a starting reference value of the subject's blood pressure is known (i.e. an offset value for the equation above), then a quantitative absolute measure of arterial blood pressure can be derived, based on adding/subtracting the calculated pressure change value from the starting reference value.

For example, an absolute blood pressure measure, BP, could be derived based on the following equation:

$$\text{BP} = \text{calibrated\_offset} + [(\text{SectionNumberNewMax} - \text{SectionNumberOldMax}) * \Delta p]$$

where calibrated_offset is a reference blood pressure measurement acquired at a start of a monitoring period, for example using an oscillometric method.

In accordance with one or more embodiments, the system may further comprise a light source adapted to direct a light output toward the tissue area of the user to which the tissue contact section 12 of the apparatus 10 is applied. This allows for more accurate or reliable PPG measurements, since the spectral properties and intensity of the light can be controlled. However, a natural light source can instead be used, meaning that provision of an artificial illumination source is not essential.

In accordance with one or more embodiments, the system may further comprise a light redirection means arranged to receive the optical emissions from the plurality of tissue contact locations, and redirect the optical emissions in an optical path toward the optical sensing device 14. For example the light redirection means may comprise one or more mirrors. The light redirection means may be advantageous in cases where there is no clear view accessible of the tissue contact area, for example if it is obscured by medical imaging apparatus such as an MRI scanner. In this case the light redirection means permits the optical output to be redirected to a location where it can be optically sensed by the optical sensing apparatus. For example, the means may comprise an arrangement of one or more mirrors, or an optical waveguide means such as a periscope.

In accordance with one or more embodiments, the system may further comprise means for monitoring one or more properties of at least one artery of the subject. For example, there may be provided means for detecting a location, size and/or compliance of one or more arteries. One or more arteries maybe arteries directly beneath the tissue contact section. By way of example, this may be achieved using infrared light to measure locations of arteries, and/or size of arteries, beneath the surface of subject's tissue, to improve positioning of the tissue contact section relative to the arteries.

By way of further explanation, location and size of arteries beneath a region of tissue can be detected with any one or more of the following methods:

- Optically, using e.g. infrared light.
- Thermally, by measuring temperature variation over a skin area using a thermal sensing means such as a thermal camera. The location of an artery underneath a skin area corresponds to a location of higher temperature at the skin surface.
- Through application of spatially distributed vibrations for stimulating dilatation of an artery beneath the skin. This can be achieved for example using a laser doppler vibrometer.
- Application of coherent light to the tissue region, and detecting locations of arteries based on a resulting inference patters (speckle pattern).

In order to realize the non-uniform pressure profile to the skin, in accordance with one or more embodiments, the tissue contact section 12 of the body-mountable apparatus 10 may comprise an optical window 22 adapted to engage with the tissue area, and wherein the optical window has a non-uniform thickness. The body-mountable apparatus 10 may comprise a retention means for holding the optical window against the tissue area with a baseline application force. As a consequence the non-uniform thickness results in non-uniform pressure.

Fig. 4 and Fig. 5 illustrate two example optical windows 22.

In each case, the optical window 22 has a contact surface 28 adapted to engage with said tissue area of the user, and wherein the optical window has a non-uniform thickness, t, in a dimension out-of-plane of said contact surface.

The body-mounted apparatus 10 may be adapted to urge the optical window against the area of the user's tissue with a force which is symmetrically or homogeneously distributed across the contact surface area. As such, the different thicknesses result in non-uniform pressure being exerted across the tissue contact area.

In the example of Fig. 4, the optical window has a stepped height profile. The optical window 22 comprises a plurality of window area sections 32, each window area section having a different respective thickness. Each discrete window section 32 has a uniform thickness across its respective area, so that the thickness varies discontinuously across the window.

In the example of Fig. 5, the optical window 22 has a continuously varying thickness, t, across at least a portion of the window area. This provides a continuous pressure gradient. The plurality of locations may be a set of locations along this pressure gradient, for instance at regular spatial intervals

Although in the above-described example, the contact pressure varies in a spatially linear line, this is not essential. The variation in applied pressure could follow any spatial pattern, e.g. varying along a curved line, varying in a circle, or varying in a 2D distribution pattern.

Window areas of different thickness may be marked so that they can be easily identified, e.g. by the optical sensing device 14. For example, the optical window may feature a plurality of optically visible markings disposed on a surface facing away from the user when the apparatus 10 is mounted to the user's head, and each optically visible marking at a location corresponding to a different window thickness. The markings may be textual markings, or could be an optically readable code such as a QR code. Each window section 32 might be marked in some examples.

The markings may be readable by the optical sensing apparatus, e.g. a readable code, to permit the optical sensing apparatus to (spatially) identify the different tissue contact locations. The markings may provide an indication of the relative pressure applied at the corresponding location.

For example, where the optical window comprises a plurality of window sections of different thicknesses, each window section may feature a respective optically visible marking.

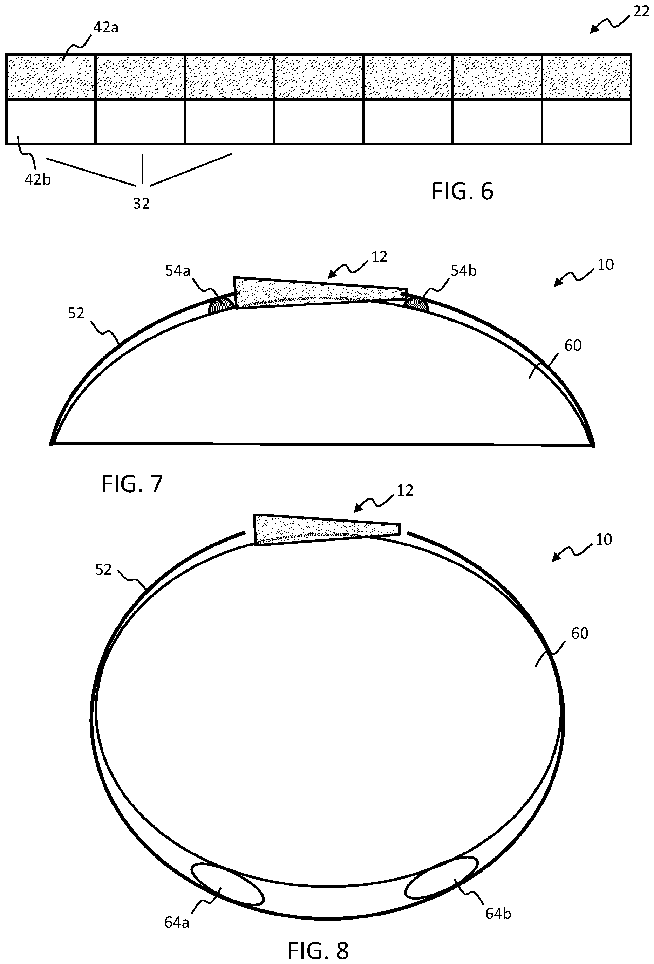

In accordance with one or more embodiments, and as illustrated in Fig. 6, where the optical window 22 comprises a plurality of window sections 32, each window section 32 may comprise two or more sub-regions 42a, 42b. One 42a of the sub-regions may comprise visible indicator means for indicating a relative or absolute pressure with which the window section 32 is being applied to the skin, and a second section 42b is optically transparent permitting the optical sensing apparatus 14 to obtain the remote PPG measurements via this sub-region.

In one or more embodiments, at least one or more regions of the optical window 22 may include a pressure-responsive material having an optical characteristic which is adapted to vary as a function of pressure. This may provide the visible indication of pressure of each window section 32. For example, a material may be used having a stress-induced change of refractive index.

By way of one example, the tissue contact section 12 may comprise an optical window 22, at least a section of which is formed of a translucent material having a color tint, and wherein said at least section of the window stretches with applied pressure, and wherein the greater the stretch, the thinner the window section becomes, and the lighter the observable color tint. Variations in pressure may therefore be detected based on detecting variations in color of the at least section of the window across the tissue contact region.

By way of further example, there is a known optical technique to make stress visible via birefringence maps. Reference is made for example to the document "TIE-27, Stress in Optical Glass", published October 2019, by Advanced Optics, Schott AG. The same effect can also be achieved in other materials such as translucent plastics.

By way of a further example, at least a section of the optical window may be provided with markings at a visible surface delineating a grid, and wherein the material is adapted to stretch laterally in response to applied normal pressure, and wherein normal pressure can be determined based on measurement of changes in distance between points on the grid. This could be measured using a camera for example.

For example, with reference to the example of Fig. 6, each first subregion 42a may be formed of, or comprise, a pressure responsive material, adapted to change a property such as a color, tint, polarization, or refractive index responsive to pressure. The second subregion 42b may be optically transparent. This enables identification of the locally applied contact pressure at each window section 32, while the second sub-region 42b can be used to measure the pulse amplitude.

In accordance with one or more embodiments, the body-mountable apparatus 10 may have means permitting adjustment of a baseline pressure with which the tissue contact section 12 is applied to the skin.

For example, Fig. 7 and Fig. 8 show two examples of a body-mountable apparatus arranged, when mounted to the body of the user, to hold the tissue-contact section 12 against the tissue of the user with an application force, and wherein the apparatus includes actuation means 54, 64 for varying said application force. The apparatus is head-mountable in this case.

Fig. 7 shows an example having actuator means either side of the tissue contact section 12. Fig. 8 shows an example with actuator means disposed between a band or strap 52 of the body-mountable apparatus 10 and the head.

Referring first to Fig. 7, this example comprises the actuation means comprising a respective actuator element 54a, 54b disposed at each of a first and second side of the skin contact section 12. In this example, the optical window 22 forms the major portion of the tissue contact section 12. The example shows a first actuator element 54a at one end of the tissue contact section, and a second actuator element 54b at a second end. The actuator elements are positioned so as to enable relative tilting of the two ends of the tissue contact section to thereby adjust a pressure differential between the first and second tissue contact sections, to thereby adjust a linear pressure gradient between the two ends. This thereby allows for varying the non-uniform pressure profile exerted across the tissue contact area by the tissue contact section of the head-mountable apparatus 10.

By way of one example, each actuator element 54a, 54b may be an electroactive polymer (EAP) actuator element. An EAP actuator element comprises an EAP material which is adapted to exhibit a deformation in response to stimulation by an electrical current or voltage. The extent of deformation is dependent on the magnitude of the voltage or the amplitude of the current.

This allows the pressure that the optical window exerts on the tissue to be remotely changed. The actuator elements 54a, 54b may be incorporated in the band 52. By placing the actuators at two opposite sides of the optical window 22, this allows "lifting" of the window with respect to the skin. By altering the height of the optical window with respect to the skin, the average pressure that the window exerts on the skin can be altered and this thereby allows alteration of a baseline or "offset" pressure.

The two actuator elements 54a, 54b may be adjusted in parallel (symmetrically) to change the baseline application pressure, or they may be adjusted non-symmetrically so that the linear pressure gradient can be adjusted by effectively tilting the optical window by a controllable degree.

Fig. 8 shows a second example wherein the body-mountable apparatus 10 comprises a band 52 for holding the tissue contact section 12 against a tissue region of a user (e.g. a headband to hold it against the forehead), and wherein the apparatus includes a pair of actuator elements 64a, 64b arranged to be disposed between the band and the user's body when the apparatus is mounted to the user, and wherein actuation of the actuator elements is adapted to vary a separation distance of the band from the body. By way of example the actuator elements in this example comprise inflatable balloons having an adjustable inflation level. This embodiment also allows the baseline or offset pressure exerted by the optical window to be adjusted by adjusting the inflation level of the balloons.

In some embodiments, the actuator elements 54a, 54b either side of the skin contact section 12 may be provided in combination with the adjustable balloons 64a, 64b.

Examples in accordance with a further aspect of the invention provide a method, comprising: simultaneously applying a different respective contact pressure to each of a plurality of tissue-contact locations across a tissue area of a user; detecting optical emissions from said tissue area of the user and generating optical sensing data based thereon; deriving a photoplethysmography (PPG) signal for each of the plurality of tissue-contact locations based on the optical sensing data; and detecting a blood pressure of the user, or a change in blood pressure of the user, based on the PPG signals for the plurality of tissue contact locations and based on the respective contact pressures at each of the tissue contact locations.

The second, third and fourth steps of the above method may be implemented by a computer or a processor.

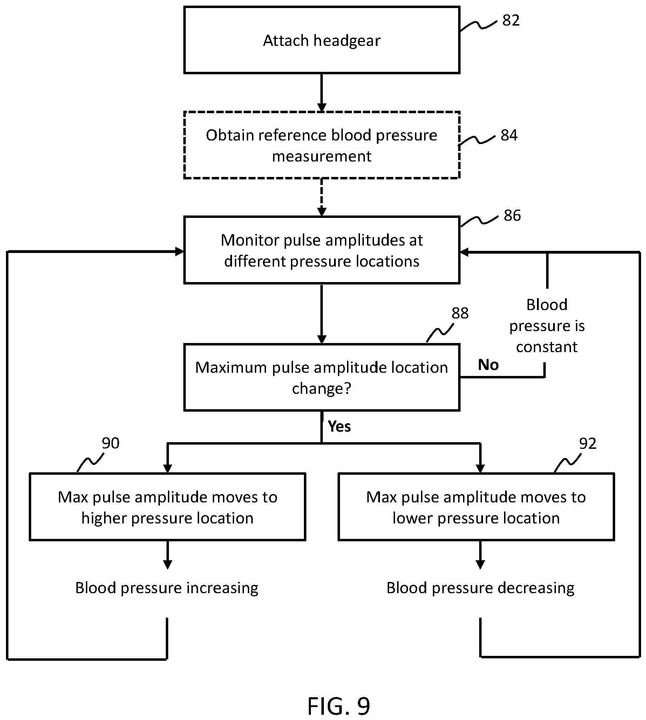

One example method in accordance with one or more embodiments is outlined in block diagram form in Fig. 9.

The method comprises a first step of applying 82 the body-mountable apparatus 10 to the body part of a subject (e.g. head). As a result of this step, a plurality of different pressures are applied at a plurality of different tissue contact locations simultaneously across an external region of tissue.

Optionally, following attachment of the apparatus, the method may comprise obtaining 84 a reference blood pressure measurement, for example using an oscillometric technique. This may be stored in a local datastore of a processor which implements the method in some examples.

The method further comprises obtaining PPG sensor readings for each of the plurality of tissue contact locations, and thereby monitoring 86 the pulse amplitude at each of the different tissue contact locations. The method further comprises identifying which of the tissue contact locations has a maximum pulse amplitude reading. The method further comprises monitoring 88 for any change in which of the tissue contact regions registers the maximum pulse amplitude. If there is no change, the method continues monitoring pulse amplitudes.

If it is detected 90 that the maximum pulse amplitude has moved to a tissue contact location corresponding to a higher application pressure, this indicates that a blood pressure of the subject is increasing. Output may be generated indicative of this determination. If it is detected 92 that the maximum pulse amplitude has moved to a tissue contact location of a lower application pressure, this indicates that the subject blood pressure is decreasing. A data output may be generated indicative of this determination.

The above method may be used by way of example during a medical imaging procedure such as an MRI or CT scan, where a critical change in patient blood pressure during the procedure should be detected, but where access to the patient is limited. The technique could also be applied in other scenarios such as emergency department waiting rooms or the general ward.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A system comprising:

    a head-mountable apparatus (10) arranged, when mounted to a user's head, to engage with a plurality of tissue contact locations across a tissue area of the user, and to apply a different respective contact pressure to each of the plurality of tissue-contact locations;
    an optical sensing device (14) adapted to detect optical emissions from the tissue area of the user and output optical sensing data based thereon; and
    a processor (16), adapted to:

        receive the optical sensing data;
        derive a photoplethysmography (PPG) signal for each of the plurality of tissue-contact locations based on the optical sensing data;
        detect a blood pressure of the user, or a change in blood pressure of the user, based on the PPG signals for the plurality of tissue contact locations and based on the respective contact pressures at each of the tissue contact locations.

2. A system as claimed in claim 1, wherein the processor is adapted to determine a pulse amplitude signal for each of the different tissue contact locations, and wherein detecting the blood pressure or change in blood pressure is based on the pulse amplitude signal for each of the tissue-contact locations.

3. A system as claimed in claim 2, wherein the processor is adapted to:

    identify which of the plurality of tissue contact locations has a highest detected pulse amplitude;
    detect a change in which of the tissue contact locations has a highest detected pulse amplitude;
    detect occurrence of a change in blood pressure based on said detected change in the tissue contact location for which the pulse amplitude is highest.

4. A system as claimed in claim 3, wherein the processor is adapted to:

    detect a change in tissue contact location for which the pulse amplitude is highest from a first location to a second location, and
    if the contact pressure of the second location is higher than the first location, detect occurrence of an increase in blood pressure, and if the contact pressure of the second location is lower than the first location, detect occurrence of a decrease in blood pressure.

5. A system as claimed in any of claims 1-4, further comprising a light source adapted to direct a light output toward said tissue area of the user.

6. A system as claimed in any of claims 1-5, wherein the head-mountable apparatus comprises a tissue contact section (12) arranged to engage with said tissue area of the user to apply the different contact pressures to each of the plurality of tissue contact locations, and wherein the tissue contact section incorporates an optical window (22) permitting optical communication with the plurality of tissue contact locations by the optical sensing device (14).

7. A system as claimed in claim 6, wherein the optical window (22) is arranged to engage with said tissue area of the user to apply the different contact pressures to each of the plurality of tissue contact locations.

8. A system as claimed in claim 7, wherein the optical window (22) has a contact surface (28) adapted to engage with said tissue area of the user, and wherein the optical window has a non-uniform thickness (t) in a dimension out-of-plane of said contact surface.

9. A system as claimed in claim 8, wherein
    the optical window (22) has a continuously varying thickness (t) across at least a portion of the window area; and/or
    the optical window (22) comprises a plurality of window area sections (32), each window area section having a different respective thickness.

10. A system as claimed in claim 8 or 9, wherein the optical window features a plurality of optically visible markings disposed on a surface facing away from the user when the apparatus (10) is mounted to the user's head, and each optically visible marking at a location corresponding to a different window thickness.

11. A system as claimed in any of claims 7-10, wherein the optical window (22) includes a pressure-responsive material having an optical characteristic which is adapted to vary as a function of pressure.

12. A system as claimed in any of claims 6-11, wherein the head-mountable apparatus (10), when mounted to the head of the user, is arranged to hold the tissue-contact section (12) against the tissue of the user with an application force, and wherein the apparatus includes actuation means (54, 64) for varying said application force.

13. A system as claimed in claim 12, wherein the apparatus (10) comprises a headband (52) for holding the tissue contact section (12) against a forehead of the user, and wherein the apparatus includes one or more actuator elements (64) arranged to be disposed between the headband and the user's head when the apparatus is mounted to the user, and wherein actuation of the actuator elements is adapted to vary a separation distance of the headband from the head.

14. A system as claimed in claim 12 or 13, wherein the actuation means comprises one or more electroactive polymer (EAP) actuator elements (54) disposed at each of a first and second side of the skin contact section (12).

15. A method, comprising:

simultaneously applying a different respective contact pressure to each of a plurality of tissue-contact locations across a tissue area of a user;
detecting optical emissions from said tissue area of the user and generating optical sensing data based thereon;
deriving a photoplethysmography (PPG) signal for each of the plurality of tissue-contact locations based on the optical sensing data;
detecting a blood pressure of the user, or a change in blood pressure of the user, based on the PPG signals for the plurality of tissue contact locations and based on the respective contact pressures at each of the tissue contact locations.

FIG. 1

FIG. 2

FIG. 3

FIG. 4

FIG. 5

FIG. 6

FIG. 7

FIG. 8

Attach headgear ⌇ 82

Obtain reference blood pressure measurement ⌇ 84

Monitor pulse amplitudes at different pressure locations ⌇ 86

Maximum pulse amplitude location change? ⌇ 88

**No**

Blood pressure is constant

**Yes**

90 ⌇ Max pulse amplitude moves to higher pressure location

92 ⌇ Max pulse amplitude moves to lower pressure location

Blood pressure increasing

Blood pressure decreasing

FIG. 9

**EUROPEAN SEARCH REPORT**

Application Number

EP 21 17 7428

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/405163 A1 (HILGERS ACHIM RUDOLF [NL] ET AL) 31 December 2020 (2020-12-31) | 15 | INV.<br>A61B5/0225<br>A61B5/00 |
| Y | * paragraph [0013] *<br>* paragraph [0020] - paragraph [0030] *<br>* paragraph [0047] *<br>* paragraph [0071] - paragraph [0079] *<br>* paragraph [0084] - paragraph [0106] *<br>* paragraph [0116] - paragraph [0119] *<br>* paragraph [0127] - paragraph [0131] *<br>* paragraph [0136] *<br>* figures 1-6 * | 1-14 | |
| A | SH SONG ET AL: "Estimation of blood pressure using Photoplethysmography on the wrist",<br>COMPUTERS IN CARDIOLOGY, 2009,<br>1 January 2009 (2009-01-01), pages 741-744, XP055424181,<br>Piscataway, NJ, USA<br>ISBN: 978-1-4244-7281-9<br>* page 741, right-hand column, last paragraph - page 743, left-hand column, paragraph 2 *<br>* figures 1-5 * | 1-15 | |
| Y | US 7 300 404 B1 (KOLLURI SAI [US] ET AL) 27 November 2007 (2007-11-27)<br>* column 2, line 50 - column 3, line 14 * | 1-14 | TECHNICAL FIELDS SEARCHED (IPC)<br><br>A61B |
| A | US 2007/055163 A1 (ASADA HARUHIKO H [US] ET AL) 8 March 2007 (2007-03-08)<br>* paragraph [0057] *<br>* paragraph [0063] *<br>* paragraph [0067] *<br>* paragraph [0075] - paragraph [0076] *<br>* paragraph [0109] *<br>* paragraph [0124] - paragraph [0125] *<br>* figures 2,4C,5 * | 1-15 | |

-/--

The present search report has been drawn up for all claims

| Place of search | Date of completion of the search | Examiner |
|---|---|---|
| Berlin | 28 October 2021 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 21 17 7428

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2015/078735 A1 (KONINKL PHILIPS NV [NL]) 4 June 2015 (2015-06-04) * page 3, line 2 - line 24 * * page 9, line 7 - page 12, line 23 * * figures 1-5 * ----- | 1-15 | |
| A | US 2019/223737 A1 (TZVIELI ARIE [US] ET AL) 25 July 2019 (2019-07-25) * paragraph [0011] * * paragraph [0053] - paragraph [0054] * * paragraph [0057] * * paragraph [0064] * * paragraph [0072] * * figures 20,21 * ----- | 1-15 | |

TECHNICAL FIELDS
SEARCHED (IPC)

The present search report has been drawn up for all claims

| Place of search | Date of completion of the search | Examiner |
|---|---|---|
| Berlin | 28 October 2021 | Völlinger, Martin |

EPO FORM 1503 03.82 (P04C01)

page 2 of 2

## ANNEX TO THE EUROPEAN SEARCH REPORT
## ON EUROPEAN PATENT APPLICATION NO.

EP 21 17 7428

This annex lists the patent family members relating to the patent documents cited in the above-mentioned European search report.
The members are as contained in the European Patent Office EDP file on
The European Patent Office is in no way liable for these particulars which are merely given for the purpose of information.

28-10-2021

| Patent document cited in search report | | Publication date | Patent family member(s) | | Publication date |
|---|---|---|---|---|---|
| US 2020405163 | A1 | 31-12-2020 | CN 111818844 A | | 23-10-2020 |
| | | | EP 3536233 A1 | | 11-09-2019 |
| | | | EP 3761868 A1 | | 13-01-2021 |
| | | | JP 2021509626 A | | 01-04-2021 |
| | | | US 2020405163 A1 | | 31-12-2020 |
| | | | WO 2019170689 A1 | | 12-09-2019 |
| US 7300404 | B1 | 27-11-2007 | CN 101138493 A | | 12-03-2008 |
| | | | DE 102007042298 A1 | | 27-03-2008 |
| | | | JP 5026201 B2 | | 12-09-2012 |
| | | | JP 2008062060 A | | 21-03-2008 |
| | | | US 7300404 B1 | | 27-11-2007 |
| US 2007055163 | A1 | 08-03-2007 | US 2007055163 A1 | | 08-03-2007 |
| | | | WO 2007024777 A2 | | 01-03-2007 |
| WO 2015078735 | A1 | 04-06-2015 | CA 2931377 A1 | | 04-06-2015 |
| | | | CN 105792742 A | | 20-07-2016 |
| | | | EP 3073905 A1 | | 05-10-2016 |
| | | | JP 6666244 B2 | | 13-03-2020 |
| | | | JP 2016539697 A | | 22-12-2016 |
| | | | US 2017164904 A1 | | 15-06-2017 |
| | | | WO 2015078735 A1 | | 04-06-2015 |
| US 2019223737 | A1 | 25-07-2019 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10290104 B2
- US 9980658 B2
- US 10242441 B2
- US 10818010 B2

**Non-patent literature cited in the description**

- **MOÇO A ; VERKRUYSSE W.** Pulse oximetry based on photoplethysmography imaging with red and green light: Calibratability and challenges. *J Clin Monit Comput.,* February 2021, vol. 35 (1), 123-133
- **VAN GASTEL, M. et al.** Data-Driven Calibration Estimation for Robust Remote Pulse-Oximetry. *Appl. Sci.,* 2019, vol. 9, 3857
- **MARK VAN GASTEL et al.** Near-continuous non-contact cardiac pulse monitoring in a neonatal intensive care unit in near darkness. *Proc. SPIE 10501, Optical Diagnostics and Sensing XVIII: Toward Point-of-Care Diagnostics,* 20 February 2018, 1050114
- **VERKRUYSSE, WIM PHD et al.** Calibration of Contactless Pulse Oximetry. *Anesthesia & Analgesia,* January 2017, vol. 124 (1), 136-145
- **AARTS LA et al.** Non-contact heart rate monitoring utilizing camera photoplethysmography in the neonatal intensive care unit - a pilot study. *Early Hum Dev.,* December 2013, vol. 89 (12), 943-8
- **SCHOTT AG.** TIE-27, Stress in Optical Glass. *Advanced Optics,* October 2019